# EUROPÄISCHE PATENTSCHRIFT

(11) **EP 2 585 549 B1**
(45) Veröffentlichungstag und Bekanntmachung des Hinweises auf die Patenterteilung: **28.02.2018**
(21) Anmeldenummer: 11735979.4
(22) Anmeldetag: 08.06.2011
(51) Int. Cl.: C09J 7/02

(54) **LUMINESZENZFÄHIGE TRENN- BZW. SCHUTZFOLIE**
LUMINESCABLE RELEASE OR PROTECTIVE FILM
FILM DE SÉPARATION OU DE PROTECTION LUMINESCENT

(30) Priorität: 22.06.2010 DE 102010024638
(43) Veröffentlichungstag der Anmeldung: 01.05.2013
(73) Patentinhaber: Infiana Germany GmbH & Co. KG, 91301 Forchheim (DE)
(72) Erfinder: SITZMANN, Stefan, 91356 Kirchehrenbach (DE); DISTLER, Georg, 91344 Waischenfeld (DE)
(74) Vertreter: Kutzenberger, Helga
(86) Internationale Anmeldenummer: PCT/EP2011/002802
(87) Internationale Veröffentlichungsnummer: WO 2011/160774

(56) Entgegenhaltungen:
- DE-T3- 69 302 981
- US-A- 4 250 382

## Beschreibung

Die vorliegende Erfindung betrifft eine Trenn- bzw. Schutzfolie, deren Silikonbeschichtung zumindest in regelmäßigen Abständen jeweils aus einem wenigstens eine lumineszenz fähige Verbindung enthaltenden, lumineszenzfähigen Silikonbeschichtungs abschnitt besteht, dadurch gekennzeichnet, dass die lumineszenzfähigen Silikonbeschichtungsabschnitte in Ausgestaltung bzw. Form dem mit der Trenn- bzw. Schutzfolie auszurüstenden Produkt entsprechen und 0,1 bis 15 Gew.-%, bezogen auf das Gesamtgewicht der Silikonbeschichtung, der lumineszenzfähigen Verbindung enthalten, ein Verfahren zur Ausrüstung von Ausgangsprodukten mit einer solchen Trenn- bzw. Schutzfolie, durch dieses Verfahren hergestellte Produkte und ferner eine Vorrichtung zur Durchführung des vorstehend genannten Verfahrens.

Trennfolien werden vielfach als abziehbare Schutzfolien für Klebebänder, Selbstklebeetiketten oder klebefähige Sanitär- und Hygieneartikel eingesetzt, um ein Verkleben dieser einseitig oder beidseitig klebenden Produkte bei der Lagerung oder Verarbeitung zu verhindern und müssen sich durch eine gute Trennwirkung gegen Klebstoffe wie z.B. druckempfindliche Klebstoffe oder andere klebefähige Materialien auszeichnen.

Aus dem Stand der Technik wie z.B. aus EP 1 277 802 A1 oder EP 0 769 540 A2 sind bereits silikonisierte Trennfolien bekannt.

Zum Aufbringen solcher mit einem Klebstoff ausgerüsteten Trennfolien auf Ausgangsprodukte wie Sanitär- oder Hygiene-Ausgangsprodukte oder umgekehrt sind üblicherweise farbige Tastmarken, d.h. farbige Markierungen in bestimmten Abständen auf der silikonisierten Fläche der Trennfolie z.B. durch Aufdrucken angbracht, die bei der Herstellung von Produkten wie z. B. Sanitär- oder Hygiene-Produkten, die eine solche Trennfolie aufweisen, in einer dafür vorgesehenen Vorrichtung eine genaue Platzierung der Trennfolien und der Ausgangsprodukte zueinander ermöglichen.

Nachteilig bei der Herstellung von mit einer solchen Trennfolie ausgestatteten Produkten ist jedoch, dass es durch die außerhalb oder auf dem Randbereich der silikonisierten Flächen der Trennfolien aufgebrachten vorzugsweise lumineszenzfähigen Tastmarken insbesondere beim Zusammenführen und Verbinden der mit einem Klebstoff ausgerüsteten Trennfolie mit dem Ausgangsprodukt zu Fehlern bei der Positionierung der klebefähigen Trennfolien und der Ausgangsprodukte zueinander und damit zu einem nicht vernachlässigbaren Ausschuss kommen kann. Zudem sind zusätzliche Produktionsschritte notwendig, da die Tastmarken üblicherweise durch Aufdrucken auf die Silikonbeschichtung der Trennfolien aufgebracht werden müssen, wodurch sich die Produktionszeit der Trennfolien erhöht. Zudem erfordert ein solcher Aufdruck den Einsatz von Druckformen und einen entsprechenden Verbrauch an Druckfarbe und damit höhere Produktionskosten.

Es besteht daher ein Bedarf an Trennfolien, die eine einfachere Herstellung von mit einer solchen Trennfolie ausgestatteten Produkten ermöglichen, wie z. B. entsprechenden Sanitär- oder Hygiene-Produkten, durch die die vorstehend genannten Nachteile umgangen werden können.

Aufgabe der vorliegenden Erfindung war es daher, eine Trenn- bzw. Schutzfolie zur Verfügung zu stellen, die eine rapportgenaue Produktion von mit solchen Trenn- bzw. Schutzfolien ausgerüsteten Produkten wie z. B. Sanitär- oder Hygiene-Produkten ermöglicht, ohne dass die Trenn- bzw. Schutzfolie, vorzugsweise in vorgegebenen Abständen außerhalb der mit einer Silikonbeschichtung versehenen Bereiche der Trenn- bzw. Schutzfolie oder auf der Silikonbeschichtung aufgebrachte lumineszenzfähige (Tast)Marken aufweist.

Diese Aufgabe wird durch die Bereitstellung einer Trenn- bzw. Schutzfolie gelöst, deren Silikonbeschichtung zumindest in regelmäßigen Abständen jeweils aus einem lumineszenzfähigen Silikonbeschichtungs-Abschnitt, vorzugsweise nur aus in regelmäßigen Abständen angeordneten, lumineszenzfähigen Silikonbeschichtungsabschnitten besteht. Die erfindungsgemäße Trenn- bzw. Schutzfolie zeichnet sich vorzugsweise dadurch aus, dass sie außer der lumineszenzfähigen Silikonbeschichtungsabschnitten keine weiteren lumineszenzfähigen Marken, insbesondere keine lumineszenz Tast-, Steuerungs- und/oder Druckmarken aufweist.

Mit Hilfe einer so ausgerüsteten Trenn- bzw. Schutzfolie kann auch ohne Tastmarken durch eine rapportgenaue Steuerung nicht nur eine exakte Positionierung der Trenn- bzw. Schutzfolie zu den Ausgangsprodukten, vorzugsweise Sanitär- oder Hygiene-Ausgangsprodukten zueinander, sondern auch die weitere Verarbeitung dieser mit der Trenn- bzw. Schutzfolie verbundenen Produkte, erreicht werden. Zudem wird durch den Einsatz der erfindungsgemäßen Trenn- bzw. Schutzfolie die Ausschussquote bei der Herstellung entsprechend ausgerüsteter Produkte deutlich verringert.

Unter dem Begriff "lumineszenzfähig" werden im Sinne der vorliegenden Erfindung zumindest die Abschnitte der Silikonbeschichtung der erfindungsgemäßen Trenn- bzw. Schutzfolie verstanden, die nach Anregung durch Energiezufuhr - wie z.B. durch Absorption von UV-Strahlung - elektromagnetische Strahlung emittieren kann, die beim Übergang von einem elektronisch angeregten Zustand in einen Zustand niedrigerer Energie wie z.B. in den Grundzustand entsteht, wobei die emittierte elektromagnetische Strahlung im Regelfall energieärmer, d.h. langwelliger als die vorher absorbierte ist. Die emittierte elektromagnetische Strahlung kann dabei eine Wellenlänge aufweisen, die im Wellenlängenbereich der UV-Strahlung, vorzugsweise in einem Wellenlängenbereich von 200 bis 380 nm, im Wellenlängenbereich des sichtbaren Lichts, vorzugsweise in einem Wellenlängenbereich von 380 bis 780 nm, besonders bevorzugt in einem Wellenlängenbereich von 400 bis 500 nm, und/oder im Wellenlängenbereich der Infrarot-Strahlung, vorzugsweise in einem Wellenlängenbereich von >780 nm, besonders bevorzugt von 780 nm bis 1 mm, liegen kann. Es können zwei Erscheinungsformen der Lumineszenz unterschieden werden, nämlich die Fluoreszenz und die Phosphoreszenz. Dem Fachmann sind diese Begriffe bekannt.

Vorzugsweise ist jeder Abschnitt der Silikonbeschichtung der erfindungsgemäßen Trenn- bzw. Schutzfolie ein fluoreszenzfähiger Silikonbeschichtungsabschnitt.

Vorzugsweise enthält jeder der lumineszenzfähigen Abschnitte der Silikonbeschichtung der erfindungsgemäßen Trenn- bzw. Schutzfolie zur Erzielung der Lumineszenz-Eigenschaften wenigstens eine lumineszenzfähige Verbindung, vorzugsweise wenigstens eine fluoreszenzfähige Verbindung, besonders bevorzugt wenigstens einen fluoreszenzfähigen optischen Aufheller.

Die erfindungsgemäß als lumineszenzfähigen Verbindungen eingesetzten Verbindungen sind vorzugsweise anorganische oder organische Verbindungen, besonders bevorzugt ausgewählt aus der Gruppe umfassend anorganische Farbstoffe, anorganische Pigmente, organische Farbstoffe und organische Pigmente, ganz besonders bevorzugt ausgewählt aus der Gruppe umfassend organische Farbstoffe und organische Pigmente, die jeweils lumineszieren, vorzugsweise fluoreszieren oder phosphoreszieren, besonders bevorzugt fluoreszieren können.

Vorzugsweise eignet sich als erfindungsgemäß eingesetzte lumineszenzfähige Verbindung wenigstens eine Verbindung, die bei Anregung mit UV-Strahlung, vorzugsweise bei Anregung mit UV-Strahlung in einem Wellenlängebereich von 200 bis 400 nm, luminesziert.

Vorzugsweise eignet sich als erfindungsgemäß eingesetzte lumineszenzfähige Verbindung wenigstens eine Verbindung, die bei Anregung mit UV-Strahlung Licht im Wellenlängenbereich der sichtbaren oder/und infraroten elektromagnetischen Strahlung emittiert, besonders bevorzugt Licht im Wellenlängenbereich der sichtbaren elektromagnetischen Strahlung, besonders bevorzugt in einem Wellenlängenbereich von 400 bis 500 nm, emittiert.

Unter dem Begriff "optischer Aufheller" wird im Sinne der vorliegenden Erfindung eine fluoreszenzfähige Verbindung oder eine Mischung unterschiedlicher fluoreszenzfähiger Verbindungen bezeichnet, die im Wellenlängenbereich der UV-Strahlung elektromagnetische Strahlung absorbiert und im Bereich des sichtbaren Lichtes, vorzugsweise in einem Wellenlängenbereich von 400 bis 500 nm, besonders bevorzugt in einem Wellenlängenbereich von 400 bis 480 nm, eine Fluoreszenzemission aufweist.

Vorzugsweise ist die lumineszenzfähige Verbindung ausgewählt aus der Gruppe umfassend Stilben-, Ethylen-, Coumarin-, Naphtalimid-, Pyrazolin-, Pyrazol-Verbindungen, wobei jede dieser Verbindungen ggf. substituiert sein kann. Derartige Verbindungen sind teilweise marktgeführte Produkte, wie z. B. Tinopal® OB der Firma Ciba oder UAA 00105 der Firma Xsys.

Die lumineszenzfähigen Abschnitte der Silikonbeschichtung der erfindungsgemäßen Trenn- bzw. Schutzfolie sind mit wenigstens einer lumineszenzfähigen Verbindung ausgerüstet.

In einer bevorzugten Ausführungsform ist eine der Oberflächenschichten der erfindungsgemäßen Trenn- bzw. Schutzfolie eine Silikonbeschichtung, die zumindest in regelmäßigen Abständen, vorzugsweise in regelmäßigen Abständen, aus einem lumineszenzfähigen Beschichtungsabschnitt besteht, d.h. eine Silikonbeschichtung ist, die zumindest in regelmäßigen Abständen, vorzugsweise in regelmäßigen Abständen, abschnittsweise aus einer lumineszenzfähigen Silikonbeschichtung besteht.

In einer weiteren bevorzugten Ausführungsform weist die erfindungsgemäße Trenn- bzw. Schutzfolie als eine ihrer Oberflächenschichten eine lumineszenzfähige Silikonbeschichtung auf, die nur aus in regelmäßigen Abständen angeordneten, lumineszenzfähigen Beschichtungsabschnitten besteht.

Jede der lumineszenzfähigen Beschichtungsabschnitte kann ganzflächig oder nur entsprechend der Form des Endproduktes gestaltet sein.

In einer bevorzugten Ausführungsform enthalten die lumineszenzfähigen Abschnitte der Silikonbeschichtung der erfindungsgemäßen Trenn- bzw. Schutzfolie 0,5 bis 10 Gew.-% bezogen auf das Gesamtgewicht der Silikonbeschichtung, wenigstens einer lumineszenzfähigen Verbindung,.

In einer weiteren bevorzugten Ausführungsform enthält die Silikonbeschichtung der erfindungsgemäßen Trenn- bzw. Schutzfolie 0,1 bis 15 Gew.-%, besonders bevorzugt 0,5 bis 10 Gew.-% bezogen auf das Gesamtgewicht der Silikonbeschichtung, wenigstens einer lumineszenzfähigen Verbindung,.

Vorzugsweise ist die Silikonbeschichtung der erfindungsgemäßen Trenn- bzw. Schutzfolie eine Silikonbeschichtung basierend auf wenigstens einem ausgehärteten Polysiloxan.

Unter dem Begriff "Polysiloxan" werden im Sinne der vorliegenden Erfindung Verbindungen verstanden, deren Polymerketten abwechselnd aus Silicium- und Sauerstoffatomen aufgebaut sind. Ein Polysiloxan basiert auf n wiederkehrenden Siloxan-Einheiten (-[Si(R₂)-O]-)ₙ, welche jeweils unabhängig voneinander mit zwei organischen Resten R disubstituiert sind, wobei R vorzugsweise jeweils für R¹ oder OR¹ steht und R¹ jeweils für einen Alkyl-Rest oder einen Aryl-Rest steht. Vorzugsweise basiert das erfindungsgemäße ausgehärtete Polysiloxan auf einer wiederkehrenden Dialkylsiloxan-Einheit oder auf einer wiederkehrenden Alkylaryl-Siloxan-Einheit. Je nachdem, wie viele Si-O-Bindungen eine einzelne Siloxan-Einheit, jeweils bezogen auf ein vierwertiges Siliciumatom, aufweist, lassen sich diese Einheiten in endständige monofunktionelle Siloxane (M) mit einer Si-O-Bindung, difunktionelle Siloxane (D) mit zwei Si-O-Bindungen, trifunktionelle Siloxane (T) mit drei Si-O-Bindungen und tetrafunktionelle Siloxane (Q) mit vier Si-O-Bindungen unterscheiden. Vorzugsweise weist das erfindungsgemäß eingesetzte Polysiloxan eine vernetzte ring- oder kettenförmige, besonders bevorzugt eine vernetzte kettenförmige Struktur auf, welche durch (D)-, (T)-, und/oder (Q)-Einheiten zu einem zwei-oder dreidimensionalen Netzwerk verknüpft ist. Die Anzahl n der wiederkehrenden Siloxan-Einheiten (-[Si(R₂)-O]-)ₙ in der Polysiloxankette wird als Polymerisationsgrad des Polysiloxans bezeichnet.

Die Silikonbeschichtung der erfindungsgemäßen Trenn- bzw. Schutzfolie basiert vorzugsweise auf wenigstens einem ausgehärteten, d.h. vernetzten Polysiloxan ausgewählt aus der Gruppe umfassend additionsvernetzte, vorzugsweise metallkatalysiert additionsvernetzte, kondensationsvernetzte, radikalisch vernetzte, kationisch vernetzte und/oder durch Feuchtigkeitseinwirkung vernetzte Polysiloxane.

Vorzugsweise basiert die Silikonbeschichtung der erfindungsgemäßen Trenn- bzw. Schutzfolie auf wenigstens einem ausgehärteten Polysiloxan, das durch thermische Aushärtung, durch Aushärtung mit elektromagnetischer Strahlung, vorzugsweise durch UV-Strahlung, oder durch Feuchtigkeitseinwirkung ausgehärtet wurde. Bevorzugt basiert die Silikonbeschichtung der erfindungsgemäßen Trenn- bzw. Schutzfolie auf wenigstens einem ausgehärteten Polysiloxan ausgewählt aus der Gruppe umfassend Polydialkylsiloxane, vorzugsweise Polydimethylsiloxane, und Polyalkylarylsiloxane, vorzugsweise Polymethylphenylsiloxane, die jeweils ausgehärtet sind.

Thermisch ausgehärtete Polysiloxane werden durch thermische Hydrosilylierung von Silan-Funktionen aufweisenden Polysiloxanen mit einer zumindest eine Kohlenstoff-Doppelbindung aufweisenden Verbindung erhalten. Bei den durch elektromagnetische Strahlung ausgehärteten Polysiloxanen ist die die Vernetzung der Polysiloxane durch elektromagnetische Strahlung, vorzugsweise durch UV-Strahlung, erfolgt. Die durch Einwirkung von Feuchtigkeit, vorzugsweise von Wasser, vernetzten Polysiloxanen werden durch eine Polykondensationsreaktion erhalten, bei der wenigstens eine Silan-Funktion und wenigstens eine Alkoxy-Gruppe oder wenigstens eine Alkoxysilan-Gruppe unter Abspaltung wenigstens eines Moleküls Alkohol eine Si-O-Bindung ausbilden. Die auszuhärtenden Polysiloxane weisen daher jeweils die für die Vernetzung benötigten miteinander reagierenden funktionellen Gruppen auf.

Die Schichtdicke der Silikonbeschichtung der erfindungsgemäßen Trenn- bzw. Schutzfolie ist vorzugsweise ≤ 5 µm, besonders bevorzugt ≤ 2 µm, ganz besonders bevorzugt im Bereich von 0,1 µm bis ≤ 1,5 µm.

Vorzugsweise weist die erfindungsgemäße Trenn- bzw. Schutzfolie eine einseitige Silikonbeschichtung, d.h. nur als eine ihrer Oberflächenschichten auf. Alternativ kann die erfindungsgemäße Trenn- bzw. Schutzfolie eine beidseitige Silikonbeschichtung aufweisen, wobei nur eine Silikonbeschichtung die lumineszenzfähigen Silikonbeschichtunges-Abschnitte aufweist.

Die erfindungsgemäße Trenn- bzw. Schutzfolie kann gegebenfalls eine oder mehrere Schichten, vorzugsweise zumindest eine Trägerschicht und/oder zumindest eine Barriereschicht, vorzugsweise eine Sauerstoffbarriereschicht, Wasserdampfbarrierschicht oder eine Ölbarriereschicht, aufweisen.

In einer bevorzugten Ausführungsform weist die erfindungsgemäße Trenn- bzw. Schutzfolie wenigstens eine Schicht, vorzugsweise wenigstens eine Schicht basierend auf wenigstens einem thermoplastischen Polymer, besonders bevorzugt wenigstens eine Trägerschicht basierend auf wenigstens einem thermoplastischen Polymer auf, die zumindest in regelmäßigen Abständen jeweils einen lumineszenzfähigen Silikonbeschichtungsabschnitt aufweist.

Zur Herstellung der Trägerschicht(en) der erfindungsgemäßen Trenn- bzw. Schutzfolie eignet sich vorzugsweise wenigstens ein thermoplastisches Polymer, besonders bevorzugt wenigstens ein thermoplastisches Polymer ausgewählt aus der Gruppe umfassend Polyolefine, Polyamide, Polyester und Copolymere aus wenigstens zwei Monomeren der genannten Polymere.

Vorzugsweise können zur Herstellung der Trägerschicht(en) thermoplastische Olefin-Homo-oder Copolymere von α,β-ungesättigten Olefinen mit 2-10 Kohlenstoffatomen wie z.B. Polyethylene (PE, insbesondere LDPE oder HDPE), Polypropylene (PP), Polybutylene (PB), Polyisobutylene (PI) oder Mischungen aus wenigstens zwei der genannten Polymere eingesetzt werden. Mit "LDPE" wird Polyethylen niedriger Dichte bezeichnet, welches eine Dichte im Bereich von 0,86-0,93 g/cm³ aufweist und sich durch einen hohen Verzweigungsgrad der Moleküle auszeichnet. Mit "HDPE" wird Polyethylen hoher Dichte bezeichnet, welches nur eine geringe Verzweigung der Molekülkette aufweist, wobei die Dichte im Bereich zwischen 0,94 und 0,97 g/cm³ liegen kann. Bevorzugte Polyolefine zur Herstellung der Trägerschicht(en) sind Ethylen-Homo- oder Copolymere und Propylen-Homo- oder Copolymere.

Soweit zur Herstellung der Trägerschicht(en) Polyamide verwendet werden, sind thermoplastische aliphatische, teilaromatische oder aromatische Polyamid Homo-oder Copolymere geeignet. Solche Polyamide sind Polyamide aus Diaminen wie aliphatischen Diaminen mit 2-10 Kohlenstoffatomen, insbesondere Hexamethylendiamin, oder aromatischen Diaminen mit 6-10 Kohlenstoffatomen, insbesondere p-Phenylendiamin und Dicarbonsäuren wie aliphatischen oder aromatischen Dicarbonsäuren mit 6-14 Kohlenstoffatomen wie z.B. Adipinsäure, Terephthalsäure oder Isoterephthalsäure. Weiterhin können die Polyamide aus Lactamen mit 4-10 Kohlenstoffatomen wie z.B. ε-Caprolactam hergestellt worden sein. Besonders geeignete Polyamide zur Herstellung der Schicht sind z.B. PA 6, PA 12, PA 66, PA 6I, PA 6T und/oder Mischungen aus wenigstens zwei der genannten Polyamide.

Als Polyester zur Herstellung der Trägerschicht(en) können thermoplastische, aliphatische, teilaromatische oder aromatische Polyester Homo-oder Copolymere eingesetzt werden. Solche Polyester leiten sich von Polyolen wie z.B. Ethylenglycol oder 1,4-Butandiol und Dicarbonsäuren oder Dicarbonsäure-Derivaten wie Adipinsäure, und/oder Terephthalsäure ab. Erfindungsgemäß können zur Herstellung der Trägerschicht(en) als Polyester auch Polycarbonate (PC) verwendet werden. Bevorzugt eignen sich Polybutylenadipat (PBA), Polyethylenterephthalat (PET), Polybutylenterephthalat (PBT) oder die entsprechenden Co-Polymere.

Zur Herstellung der Barriereschicht(en) der erfindungsgemäßen Trenn- bzw. Schutzfolie eignet sich vorzugsweise wenigstens ein thermoplastisches Polymer, besonders bevorzugt wenigstens ein thermoplastisches Polymer ausgewählt aus der Gruppe umfassend Polyamide, Ethylen-Vinylalkohol-Copolymere (EVOH), Polyvinylalkohole (PVOH) und eine Mischung aus wenigstens zwei der genannten Polymere.

Die zur Herstellung der Barriereschicht(en) eingesetzten Polyamide (PA) sind vorzugsweise dieselben Polyamide, die auch zur Herstellung der Trägerschicht(en) der erfindungsgemäßen Trenn- bzw. Schutzfolie eingesetzt werden können.

Die zur Herstellung der Barriereschicht(en) eingesetzten Polyvinylalhohole werden durch vollständige oder unvollständige Hydrolyse von entsprechenden Polyvinylacetaten (PVA) gewonnen und umfassen somit sowohl teilverseifte Polyvinylacetate, die einen Verseifungsgrad von 50 bis 98 mol-% aufweisen, als auch vollverseifte Polyvinylacetate mit einem Verseifungsgrad ≥ 98 %.

Die zur Herstellung der Barriereschicht(en) eingesetzten Ethylen-Vinylalkohol-Copolymere (EVOH) werden durch vollständige oder unvollständige Hydrolyse von entsprechenden ethylenhaltigen Polyvinylacetaten (EVAc) gewonnen und umfassen vor allem vollverseifte ethylenhaltige Polyvinylacetate mit einem Verseifungsgrad ≥ 98 %.

Die Silikonbeschichtung sowie ggf. die Trägerschicht(en) und/oder Barriereschicht(en) der erfindungsgemäßen Trenn- bzw. Schutzfolie können, wenn notwendig, jeweils unabhängig voneinander, mit Zusatzstoffen ausgewählt aus der Gruppe umfassend Antiblockmittel, Antistatika, Antifogmittel, antimikrobielle Wirkstoffe, Farbstoffe, Farbpigmente, Stabilisierungsmittel, vorzugsweise HitzeStabilisatoren, Prozess-Stabilisatoren, Prozesshilfsmittel, Flammschutzmittel, Nukleierungsmittel, Kristallisationsmittel, vorzugsweise Kristallkeimbildner, Gleitmittel, optische Aufheller, Flexibilisierungsmittel, Siegelmittel, Weichmacher, Abstandshalter, Füllstoffe, Peel-Additive, Wachse, Benetzungsmittel, oberflächenaktive Verbindungen, vorzugsweise Tenside, und Dispergiermittel, dotiert sein. Dabei muss die Trennwirkung der Silikonbeschichtung und deren Lumineszenzfähigkeit erhalten bleiben.

Die Silikonbeschichtung sowie ggf. die Trägerschicht(en) und/oder Barriereschicht(en) der erfindungsgemäßen Trenn- bzw. Schutzfolie können, wenn notwendig, jeweils unabhängig voneinander wenigstens 0,01-30 Gew.-%, vorzugsweise wenigstens 0,1-20 Gew.-%, jeweils bezogen auf das Gesamtgewicht einer einzelnen Schicht, wenigstens einen der vorstehend genannten Zusatzstoffe enthalten.

Ein weiterer Gegenstand der vorliegenden Erfindung ist ein Verfahren zur Herstellung der erfindungsgemäßen Trenn- bzw. Schutzfolie.

Die Herstellung der erfindungsgemäßen Trenn- bzw. Schutzfolie kann nach bekannten Herstellungsverfahren wie z.B. Laminierung oder (Co)-Extrusion, vorzugsweise durch Co-Extrusion erfolgen. Dabei können einzelne ggf. vorhandene Trägerschicht(en) und/oder Barriereschicht(en) der erfindungsgemäßen Trenn- bzw. Schutzfolie durch Extrusion, vorzugsweise durch Flachfolien-Extrusion (Cast-Extrusion) oder Blasfolien-Extrusion, insbesondere durch Flachfolien-Co-Extrusion (Cast-Co-Extrusion) oder Blasfolien-Co-Extrusion gebildet werden.

In einer bevorzugten Ausführungsform können die Trägerschicht(en) und/oder ggf. vorhandenen Barriereschicht(en) der erfindungsgemäßen Trenn- bzw. Schutzfolie als ggf. mehrschichtiger Folienverbund in Form einer Schlauchfolie produziert und verarbeitet werden. Der so erhaltene gesamte ggf. mehrschichtige Folienverbund in Form einer Schlauchfolie kann direkt im Anschluss ohne vorherige Lagerung oder alternativ schon bei seiner Herstellung geprägt werden. Vorzugsweise wird der ggf. mehrschichtige Folienverbund in Form einer Schlauchfolie schon bei seiner Herstellung geprägt.

In einer weiteren bevorzugten Ausführungsform können die Trägerschicht(en) und/oder ggf. vorhandene(n) Barriereschicht(en) der erfindungsgemäßen Trenn- bzw. Schutzfolie als ggf. mehrschichtiger Cast-Folienverbund produziert und verarbeitet werden. Der so erhaltene gesamte ggf. mehrschichtige Folienverbund in Form einer Schlauchfolie kann direkt im Anschluss ohne vorherige Lagerung oder alternativ schon bei seiner Herstellung geprägt werden. Vorzugsweise wird der ggf. mehrschichtige Folienverbund in Form einer Schlauchfolie schon bei seiner Herstellung geprägt.

Vorzugsweise wird wenigstens eine Schicht, besonders bevorzugt wenigstens eine Trägerschicht, oder ein Schichtverbund aus wenigstens einer Trägerschicht und/oder wenigstens einer Barriereschicht, auf einer Oberfläche (im Fall des Schichtverbundes vorzugsweise auf der Oberfläche einer Trägerschicht des Schichtverbunds) mit einer Silikonbeschichtung ausgerüstet, die zumindest in regelmäßigen Abständen jeweils aus einem lumineszenzfähigen Abschnitt besteht. Dabei wird wenigstens eine Schicht oder der vorstehende Schichtverbund auf einer Oberfläche zumindest in regelmäßigen Beschichtungsabschnitten, vorzugsweise nur in regelmäßigen Abständen mit einer vorzugsweise flüssigen Mischung aus wenigstens einem noch nicht ausgehärteten Polysiloxan und aus wenigstens einer lumineszenzfähigen Verbindung sowie ggf. wenigstens einem vorstehend genannten Zusatzstoff ausgerüstet. Die Ausrüstung erfolgt dabei durch Beschichtung einer Oberfläche dieser Schicht oder dieses vorstehenden Schichtverbunds mit der Mischung in regelmäßigen Abständen oder ggf. abwechselnd mit einer lumineszenzfreien Beschichtungsmasse über die gesamte Oberfläche der fortlaufenden Folienbahn, vorzugsweise in regelmäßigen Abständen. Dabei kann die vorstehend genannte vorzugsweise flüssige Mischung in regelmäßigen Abständen ganzflächig, d. h. über die gesamte Folienbreite oder nur entsprechend einem Druckklischee auf der Folienbahn aufgetragen werden. Die Ausgestaltung bzw. Form der lumineszenzfähigen Abschnitte der Silikonbeschichtung kann dabei vorzugsweise an die Form des mit der Trenn- bzw. Schutzfolie auszurüstenden Ausgangsproduktes angepasst und das Druckklischee je nach Bedarf dementsprechend geändert werden. Vorzugsweise weisen daher alle lumineszenzfähigen Abschnitte der Silikonbeschichtung einer Produktionscharge dieselbe Ausgestaltung bzw. Form auf. Die Aushärtung der Mischung aus noch nicht ausgehärteten Polysiloxan und aus der lumineszenfähigen Verbindung oder der Mischung aus noch nicht ausgehärteten Polysiloxan, der lumineszenfähigen Verbindung und ggf. eines vorstehend genannten Zusatzstoffes und ggf. einer lumineszenzfreien Silikonbeschichtungsmasse erfolgt vorzugsweise durch Einwirkung von Wärme oder mit Hilfe von elektromagnetischer Strahlung, vorzugsweise UV-Strahlung, oder durch Feuchtigkeit, ggf. durch Zusatz wenigstens eines UV-Initiators und/oder eines Radikalstarters in das nicht ausgehärtete Polysiloxans oder in die Mischung, besonders bevorzugt durch Einwirkung von Wärme oder von elektromagnetischer Strahlung, vorzugsweise UV-Strahlung. Die so ausgerüstete erfindungsgemäße Trenn- bzw. Schutzfolie kann ggf. im Anschluss auf die gewünschten Maße zugeschnitten werden.

Die erfindungsgemäße Trenn- bzw. Schutzfolie ist vorzugsweise geprägt.

Die erfindungsgemäße Trenn- bzw. Schutzfolie kann mit einer nicht lumineszierenden Bedruckung ausgerüstet und/oder farbig und/oder transparent sein.

Die erfindungsgemäße Trenn- bzw. Schutzfolie zeichnet sich durch eine ausgezeichnete Haptik und verbesserte optische Eigenschafte gegenüber den bekannten Trenn- bzw. Schutzfolien.

Die erfindungsgemäße Trenn- bzw. Schutzfolie eignet sich zur Ausrüstung von vorzugsweise flächenförmigen Ausgangsprodukten, vorzugsweise entsprechenden Sanitär- oder Hygiene-Ausgangsprodukten. Vorzugsweise weisen dazu alle lumineszenzfähigen Abschnitte der Silikonbeschichtung der erfindungsgemäßen Trenn- bzw. Schutzfolie dieselbe Ausgestaltung bzw. Form auf. Vorzugsweise ist diese Ausgestaltung bzw. Form an die Ausgestaltung bzw. Form der mit einer erfindungsgemäßen Trenn- bzw. Schutzfolie auszurüstenden, vorzugsweise flächenförmgen Ausgangsprodukte, vorzugsweise entsprechenden Sanitär- oder Hygiene-Ausgangsprodukte, angepasst.

Ein weiterer Gegenstand der vorliegenden Erfindung ist ein vorzugsweise kontinuierliches Verfahren zur Ausrüstung von vorzugsweise flächenförmigen Ausgangsprodukten, vorzugsweise entsprechenden Sanitär- oder Hygiene-Ausgangsprodukten, mit einer erfindungsgemäßen Trenn- bzw. Schutzfolie.

Das erfindungsgemäße Verfahren zur Ausrüstung von vorzugsweise flächenförmigen Ausgangsprodukten, vorzugsweise entsprechenden Sanitär- oder Hygiene-Ausgangsprodukten, mit einer erfindungsgemäßen Trenn- bzw. Schutzfolie umfasst wenigstens die Schritte
(a) synchronisierte, getrennte Zuführung vereinzelter Ausgangsprodukte, vorzugsweise Sanitär- oder Hygiene-Ausgangsprodukte, und einer Trenn- bzw. Schutzfolie, deren Silikonbeschichtung zumindest in regelmäßigen Abständen jeweils aus einem lumineszenzfähigen Silikonbeschichtungsabschnitt, ggf. jeweils abwechselnd mit einem nicht-lumineszenzfähigen Silikonbeschichtungs-Abschnitt, vorzugsweise nur aus in regelmäßigen Abständen angeordneten lumineszenzfähigen Silikonbeschichtungsabschnitten besteht, die vorzugsweise in ihrer Gestaltung und Form dem damit auszurüstenden Produkt entsprechen,
(b) zumindest teilweise Ausrüstung jeder dieser lumineszenzfähigen Abschnitte der Silikonbeschichtung der Trenn- bzw. Schutzfolie mit einem vorzugsweise druckempfindlichen Klebstoff, vorzugsweise in Form von wenigstens einem Streifen, oder zumindest teilweise Ausrüstung jedes der vereinzelten Ausgangsprodukte, vorzugsweise Sanitär- oder Hygiene-Ausgangsprodukte mit einem vorzugsweise druckempfindlichen Klebstoff, vorzugsweise in Form von wenigstens einem Streifen,
(c) Zusammenführen und Verbinden jeweils eines zumindest teilweise mit einem vorzugsweise druckempfindlichen Klebstoff ausgerüsteten lumineszenzfähigen Abschnitts der Silikonbeschichtung der Trenn- bzw. Schutzfolie mit einem vereinzelten Ausgangsprodukt, vorzugsweise einem Sanitär- oder Hygiene-Ausgangsprodukt, oder Zusammenführen und Verbinden jeweils eines zumindest teilweise mit einem vorzugsweise druckempfindlichen Klebstoff ausgerüsteten vereinzelten Ausgangsproduktes, vorzugsweise Sanitär- oder Hygiene-Ausgangsproduktes mit jeweils einem lumineszenzfähigen Abschnitt der Silikonbeschichtung der Trenn- bzw. Schutzfolie,
(d) ggf. Weiterbeförderung der mit der Trenn- bzw. Schutzfolie verbundenen Produkte, vorzugsweise Sanitär- oder Hygiene-Produkte,
(e) ggf. Falten, ggf. Versiegeln und Vereinzeln und der mit der Trenn- bzw. Schutzfolie ausgestatteten Produkte, vorzugsweise Sanitär- oder Hygiene-Produkte,
(f) ggf. Verpacken jedes mit einer Trenn- bzw. Schutzfolie ausgestatteten Produktes, vorzugsweise Sanitär- oder Hygiene-Produktes, und ggf. weitere Vereinzelung der verpackten Produkte, vorzugsweise Sanitär- oder Hygiene-Produkte,
wobei zumindest die Schritte (b) und (c) und ggf. auch der Schritt (a) von wenigstens einem Lumineszenz, vorzugsweise Fluoreszenz, erfassenden Sensorelement rapportgenau gesteuert werden.

Als im Schritt (b) des erfindungsgemäßen Verfahrens eingesetzte Klebstoffe eignen sich vorzugsweise druckempfindliche Klebstoffe, besonders bevorzugt übliche, dem Fachmann bekannte vorzugsweise druckempfindliche Haftschmelzklebstoffe wie z.B. Haftschmelzklebstoffe auf Basis thermoplastischen Kautschuks. Besonders bevorzugt werden Haftschmelzklebstoffe im Schritt (b) des erfindungsgemäßen Verfahrens eingesetzt.

Vorzugsweise wird im Schritt (b) jeder der lumineszenzfähigen Abschnitte der Silikonbeschichtung der erfindungsgemäßen Trenn- bzw. Schutzfolie oder jedes der Ausgangsprodukte, vorzugsweise Sanitär- oder Hygiene-Ausgangsprodukte, zumindest teilweise mit wenigstens einem vorzugsweise druckempfindlichen Klebstoff, vorzugsweise in Form wenigstens eines Streifens, ausgerüstet.

In einer bevorzugten Ausführungsform des erfindungsgemäßen Verfahrens erfolgt im Schritt (b) eine zumindest teilweise Ausrüstung jedes der lumineszenzfähigen Abschnitte der Silikonbeschichtung der Trenn- bzw. Schutzfolie mit einem vorzugsweise druckempfindlichen Klebstoff, und im Schritt (c) ein Zusammenführen und Verbinden jeweils eines zumindest teilweise mit einem vorzugsweise druckempfindlichen Klebstoff ausgerüsteten, d.h. klebefähigen, lumineszenzfähigen Abschnitts der Silikonbeschichtung der Trenn- bzw. Schutzfolie mit einem vereinzelten Ausgangsprodukt, vorzugsweise einem Sanitär- oder Hygiene-Ausgangsprodukt.

In einer weiteren bevorzugten Ausführungsform des erfindungsgemäßen Verfahrens erfolgt im Schritt (b) eine zumindest teilweise Ausrüstung jedes der vereinzelten Ausgangsprodukte, vorzugsweise Sanitär- oder Hygiene-Ausgangsprodukte, mit einem vorzugsweise druckempfindlichen Klebstoff, und im Schritt (c) ein Zusammenführen und Verbinden jeweils eines zumindest teilweise mit einem vorzugsweise druckempfindlichen Klebstoff ausgerüsteten, d.h. klebefähigen, vereinzelten Ausgangsproduktes, vorzugsweise Sanitär- oder Hygiene-Ausgangsproduktes, mit jeweils einem lumineszenzfähigen Abschnitt der Silikonbeschichtung einer erfindungsgemäßen Trenn- bzw. Schutzfolie.

Im Schritt (e) erfolgt ggf. ein Falten und ggf. unter Versiegeln ein Vereinzeln der mit der erfindungsgemäßen Trenn- bzw. Schutzfolie ausgestatteten und ggf. darin verpackten Produkte, vorzugsweise Sanitär- oder Hygiene-Produkte. Die Vereinzelung kann durch geeignete Perforierung, Schneiden und/oder Stanzen (Aus- oder Durchstanzen) der mit der erfindungsgemäßen Trenn- bzw. Schutzfolie ausgestatteten Pordukte, vorzugsweise Sanitär- oder Hygiene-Produkte, erreicht werden. Erfolgt die Vereinzelung durch Stanzen, so werden die mit der erfindungsgemäßen Trenn- bzw. Schutzfolie ausgestatteten Produkten, vorzugsweise Sanitär- oder Hygiene-Produkte dabei vorzugsweise kontinuierlich in eine Stanzanlage geführt und unter den Stanzkopf des Stanzwerkzeuges positioniert. Mit Hilfe des Stanzstempels des Stanzwerkzeugs werden die mit der erfindungsgemäßen Trenn- bzw. Schutzfolie ausgestatteten Produkte, vorzugsweise Sanitär- oder Hygiene-Produkte mit einer einstellbaren Geschwindigkeit (Stanzgeschwindigkeit) ausgestanzt oder perforiert.

Als wenigstens ein Lumineszenz, vorzugsweise Fluoreszenz, erfassendes Sensorelement, das zumindest die Schritte (b) und (c) und ggf. auch den Schritt (a), vorzugsweise die Schritte (a) bis (c), rapportgenau steuert, eignet sich vorzugsweise jedes übliche Lumineszenz, vorzugsweise Fluoreszenz, erfassendes Sensorelement, auf UV-Strahlungsbasis, besonders bevorzugt jeder übliche Lumineszenztaster, ganz besonders bevorzugt jeder übliche Fluoreszenztaster. Ein solcher Lumineszenztaster ist z.B. der Lumineszenztaster LUT3-820 der Baureihe LUT3-8 der Firma SICK.

Ein weiterer Gegenstand der vorliegenden Erfindung ist ein vorzugsweise flächenförmiges Produkt, vorzugsweise entsprechendes Sanitär- oder Hygiene-Produkt, das mit einer erfindungsgemäße Trenn- bzw. Schutzfolie ausgerüstet ist und nach einem erfindungsgemäßen Verfahren erhältlich ist.

Vorzugsweise ist das erfindungsgemäße Sanitär- oder Hygiene-Produkt ausgewählt aus der Gruppe umfassend Damenbinden und Slip-Einlagen.

Ein weiterer Gegenstand der vorliegenden Erfindung ist eine Vorrichtung zur Durchführung des erfindungsgemäßen Verfahrens.

Die erfindungsgemäße Vorrichtung zur Durchführung des erfindungsgemäßen Verfahrens umfasst dabei wenigstens
(1) ein erstes Fördermittel zur Beförderung des vereinzelten Ausgangsproduktes, vorzugsweise Sanitär- oder Hygiene-Ausgangsprodukts,
(2) ein zweites Fördermittel zur Beförderung einer erfindungsgemäßen Trenn- bzw. Schutzfolie, deren Silikonbeschichtung zumindest in regelmäßigen Abständen jeweils aus einem lumineszenzfähigen Abschnitt, und ggf. jeweils abwechselnd aus einem nicht-lumineszenzfähigen Silikonbeschichtungsabschnitt, vorzugsweise nur aus in regelmäßigen Abständen angeordneten lumineszenzfähigen Silikonbeschichtungsabschnitten besteht,
(3) eine Dosiereinrichtung zur zumindest teilweisen Ausrüstung jeder der lumineszenzfähigen Abschnitte der Silikonbeschichtung der Trenn- bzw. Schutzfolie mit einem vorzugsweise druckempfindlichen Klebstoff, oder zur zumindest teilweisen Ausrüstung jedes vereinzelten Ausgangsproduktes, vorzugsweise Sanitär- oder Hygiene-Ausgangsprodukts mit einem vorzugsweise druckempfindlichen Klebstoff,
(4) eine Umlenkvorrichtung zum Zusammenführen des ersten Fördermittels mit dem zweiten Fördermittel und zum Verbinden jeder zumindest teilweise mit einem vorzugsweise druckempfindlichen Klebstoff ausgerüsteten lumineszenzfähigen Abschnitte der Silikonbeschichtung der Trenn- bzw. Schutzfolie mit einem vereinzelten Ausgangsprodukt, vorzugsweise Sanitär- oder Hygiene-Ausgangsprodukt, oder zum Verbinden jedes der zumindest teilweise mit einem vorzugsweise druckempfindlichen Klebstoff ausgerüsteten, vereinzelten Ausgangsprodukte, vorzugsweise Sanitär- oder Hygiene-Ausgangsprodukt, mit jeweils einem lumineszenzfähigen Abschnitt der Silikonbeschichtung einer erfindungsgemäßen Trenn- bzw. Schutzfolie,
(5) ggf. eine Faltstation zum Falten jedes Produktes, vorzugsweise jedes Sanitär- oder Hygiene-Produktes, und,
(6) ggf. eine Siegel-, und eine Stanz- und/oder Schneidevorrichtung zum Vereinzeln der Produkte, vorzugsweise Sanitär- oder Hygiene-Produkte, und,
(7) ggf. eine Verpackungsstation zur Verpackung jedes Produktes, vorzugsweise jedes Sanitär- oder Hygiene-Produktes,
(8) und ein Lumineszenz, vorzugsweise Fluoreszenz empfindliches Sensorelement in direktem oder indirektem Kontakt mit
(9) einer Steuereinheit zur rapportgenauen Steuerung zumindest der beiden Fördermittel, der Dosiereinrichtung, der Umlenkvorrichtung, der Stanz- und/oder Schneidevorrichtung und ggf. der Verpackungsstation.

In einer bevorzugten Ausführungsform der erfindungsgemäßen Vorrichtung ist die Dosiereinrichtung (3) eine Dosiereinrichtung zur zumindest teilweisen Ausrüstung jeder der lumineszenzfähigen Abschnitte der Silikonbeschichtung der Trenn- bzw. Schutzfolie mit einem vorzugsweise druckempfindlichen Klebstoff, und die Umlenkvorrichtung (4) eine Umlenkvorrichtung zum Zusammenführen des ersten Fördermittels mit dem zweiten Fördermittel und zum Verbinden jeder zumindest teilweise mit einem vorzugsweise druckempfindlichen Klebstoff ausgerüsteten lumineszenzfähigen Abschnitte der Silikonbeschichtung der Trenn- bzw. Schutzfolie mit einem vereinzelten Ausgangsprodukt, vorzugsweise Sanitär- oder Hygiene-Ausgangsprodukt.

In einer weiteren bevorzugten Ausführungsform der erfindungsgemäßen Vorrichtung ist die Dosiereinrichtung (3) eine Dosiereinrichtung zur zumindest teilweisen Ausrüstung jedes vereinzelten Ausgangsproduktes, vorzugsweise Sanitär- oder Hygiene-Ausgangsprodukts mit einem vorzugsweise druckempfindlichen Klebstoff, und die Umlenkvorrichtung (4) eine Umlenkvorrichtung zum Zusammenführen des ersten Fördermittels mit dem zweiten Fördermittel und zum Verbinden jedes der zumindest teilweise mit einem vorzugsweise druckempfindlichen Klebstoff ausgerüsteten, vereinzelten Ausgangsprodukte, vorzugsweise Sanitär- oder Hygiene-Ausgangsprodukt, mit jeweils einem lumineszenzfähigen Abschnitt der Silikonbeschichtung, vorzugsweise in einer Ausgestaltung und Form, die der Ausgestaltung und Form des auszurüstenden Ausgangsprodukts entspricht, einer erfindungsgemäßen Trenn- bzw. Schutzfolie.

## Patentansprüche

1. Eine Trenn- bzw. Schutzfolie, deren Silikonbeschichtung zumindest in regelmäßigen Abständen jeweils aus einem wenigstens eine lumineszenzfähige Verbindung enthaltenden, lumineszenzfähigen Silikonbeschichtungsabschnitt besteht, **dadurch gekennzeichnet, dass** die lumineszenzfähigen Silikonbeschichtungsabschnitte in Ausgestaltung bzw. Form dem mit der Trenn- bzw. Schutzfolie auszurüstenden Produkt entsprechen und 0,1 bis 15 Gew.-%, bezogen auf das Gesamtgewicht der Silikonbeschichtung, der lumineszenzfähigen Verbindung enthalten.

2. Eine Trenn- bzw. Schutzfolie nach Anspruch 1, **dadurch gekennzeichnet, dass** die Silikonbeschichtung nur aus den in regelmäßigen Abständen angeordneten wenigstens eine lumineszenzfähige Verbindung enthaltenden, lumineszenzfähigen Silikon-Beschichtungsabschnitten besteht.

3. Eine Trenn- bzw. Schutzfolie nach Anspruch 1 oder 2, **dadurch gekennzeichnet, dass** jeder der wenigstens eine lumineszenzfähige Verbindung enthaltenden, lumineszenzfähigen Abschnitte der Silikonbeschichtung ein fluoreszenzfähiger Silikon-Abschnitt ist.

4. Eine Trenn- bzw. Schutzfolie nach einem der vorstehenden Ansprüche, **dadurch gekennzeichnet, dass** jeder der lumineszenzfähigen Silikon-Abschnitte der Silikonbeschichtung wenigstens eine fluoreszenzfähige Verbindung, besonders bevorzugt wenigstens einen fluoreszenzfähigen, optischen Aufheller, als lumineszenzfähige Verbindung enthält.

5. Eine Trenn- bzw. Schutzfolie nach einem der vorstehenden Ansprüche, **dadurch gekennzeichnet, dass** eine der Oberflächen der Trenn- bzw. Schutzfolie eine Silikonbeschichtung aufweist, die zumindest in regelmäßigen Abständen aus einem wenigstens eine lumineszenzfähige Verbindung enthaltenden, lumineszenzfähigen Silikon-Beschichtungsabschnitt und ggf. jeweils abwechselnd aus einem nicht-lumineszenzfähigen Silikon-Beschichtungsabschnitt oder nur aus in regelmäßigen Abschnitten angeordneten, wenigstens eine lumineszenzfähige Verbindung enthaltenden, lumineszenzfähigen Beschichtungsabschnitten besteht.

6. Eine Trenn- bzw. Schutzfolie nach einem der vorstehenden Ansprüche, **dadurch gekennzeichnet, dass** die lumineszenzfähigen Abschnitte der Silikonbeschichtung 0,5 bis 10 Gew.-%, bezogen auf das Gesamtgewicht der Silikonbeschichtung, der lumineszenzfähigen Verbindung enthalten.

7. Eine Trenn- bzw. Schutzfolie nach einen der vorstehenden Ansprüche, **dadurch gekennzeichnet, dass** die Trenn- und Schutzfolie außer den wenigstens eine lumineszenzfähige Verbindung enthaltenden, lumineszenzfähigen Silikonbeschichtungsabschnitten keine anderen lumineszenzfähigen Marken, insbesondere keine Tast-, Steuerungs- und/oder Druckmarken aufweist.

8. Ein Verfahren zur Ausrüstung eines vorzugsweise flächenförmigen Ausgangsproduktes, vorzugsweise eines Sanitär- oder Hygiene-Ausgangsproduktes, mit einer Trenn- bzw. Schutzfolie nach einem der Ansprüche 1 bis 7 umfassend wenigstens die Schritte
(a) synchronisierte, getrennte Zuführung vereinzelter Ausgangsprodukte, vorzugsweise Sanitär- oder Hygiene-Ausgangsprodukte, und der Trenn- bzw. Schutzfolie,
(b) zumindest teilweise Ausrüstung jedes dieser wenigstens eine lumineszenzfähige Verbindung enthaltenden, lumineszenzfähigen Silikonbeschichtungsabschnitte der Trenn- bzw. Schutzfolie mit einem vorzugsweise druckempfindlichen Klebstoff, vorzugsweise in Form von wenigstens einem Streifen, oder zumindest teilweise Ausrüstung jedes der vereinzelten Ausgangsprodukte, vorzugsweise Sanitär- oder Hygiene-Ausgangsprodukte mit einem vorzugsweise druckempfindlichen Klebstoff, vorzugsweise in Form von wenigstens einem Streifen;
(c) Zusammenführen und Verbinden jeweils eines zumindest teilweise mit einem vorzugsweise druckempfindlichen Klebstoff ausgerüsteten wenigstens eine lumineszenzfähige Verbindung enthaltenden, lumineszenzfähigen Silikonbeschichtungsabschnitts der Trenn- bzw. Schutzfolie mit jeweils einem Ausgangsprodukt, vorzugsweise einem Sanitär- oder Hygiene-Ausgangsprodukt, oder Zusammenführen und Verbinden jeweils eines zumindest teilweise mit einem vorzugsweise druckempfindlichen Klebstoff ausgerüsteten, vereinzelten Ausgangsproduktes, vorzugsweise Sanitär- oder Hygiene-Ausgangsproduktes, mit jeweils einem wenigstens eine lumineszenzfähige Verbindung enthaltenden, lumineszenzfähigen Silikonbeschichtungsabschnitt der Trenn- bzw. Schutzfolie,
(d) ggf. Weiterbeförderung der mit der Trenn- bzw. Schutzfolie verbundenen Produkte, vorzugsweise Sanitär- oder Hygiene-Produkte,
(e) ggf. Falten, ggf. Versiegeln, und Vereinzeln der mit der Trenn- bzw. Schutzfolie ausgestatteten Produkte, vorzugsweise Sanitär- oder Hygiene-Produkte,
(f) ggf. Verpacken jedes mit einer Trenn- bzw. Schutzfolie ausgestatteten Produktes, vorzugsweise Sanitär- oder Hygiene-Produktes, und ggf. weitere Vereinzelung der verpackten Produkte, vorzugsweise Sanitär- oder Hygiene-Produkte,
wobei zumindest die Schritte (b) und (c) und ggf. auch der Schritt (a) von wenigstens einem Lumineszenz, vorzugsweise Fluoreszenz, erfassenden Sensorelement rapportgenau gesteuert werden.

9. Ein Verfahren nach Anspruch 8, **dadurch gekennzeichnet, dass** das Verfahren kontinuierlich ist.

10. Ein vorzugsweise flächenförmiges Produkt, vorzugsweise ein Sanitär- oder Hygieneprodukt, erhältlich nach einem Verfahren gemäß Anspruch 8 oder 9.

11. Ein Sanitär- oder Hygiene-Produkt nach Anspruch 10, **dadurch gekennzeichnet, dass** das Sanitär- oder Hygiene-Produkt eine Damenbinde oder eine Slip-Einlage ist.

12. Eine Vorrichtung zur Durchführung eines Verfahrens nach Anspruch 8 oder 9, wenigstens umfassend
(1) ein erstes Fördermittel zur Beförderung der vereinzelten Ausgangsprodukte, vorzugsweise Sanitär- oder Hygiene-Ausgangsprodukte,
(2) ein zweites Fördermittel zur Beförderung einer Trenn- bzw. Schutzfolie nach einem der Ansprüche 1-7, deren Silikonbeschichtung zumindest in regelmäßigen Abständen jeweils aus einem wenigstens eine lumineszenzfähige Verbindung enthaltenden, lumineszenzfähigen Silikonbeschichtungsabschnitt besteht und die lumineszenzfähigen Silikonbeschichtungsabschnitte in Ausgestaltung bzw. Form dem mit der Trenn- bzw. Schutzfolie auszurüstenden Produkt entsprechen und 0,1 bis 15 Gew.-%, bezogen auf das Gesamtgewicht der Silikonbeschichtung, der lumineszenzfähigen Verbindung enthalten,
(3) eine Dosiereinrichtung zur zumindest teilweisen Ausrüstung von jedem der lumineszenzfähigen Silikonbeschichtungsabschnitt mit einem vorzugsweise druckempfindlichen Klebstoff, oder zur zumindest teilweisen Ausrüstung jedes vereinzelten Ausgangsproduktes, vorzugsweise Sanitär- oder Hygiene-Ausgangsprodukts mit einem vorzugsweise druckempfindlichen Klebstoff,
(4) eine Umlenkvorrichtung zum Zusammenführen des ersten Fördermittels mit dem zweiten Fördermittel und zum Verbinden von jedem zumindest teilweise mit einem vorzugsweise druckempfindlichen Klebstoff ausgerüsteten wenigstens eine lumineszenzfähige Verbindung enthaltenden, lumineszenzfähigen Silikonbeschichtungsabschnitt mit einem vereinzelten Ausgangsprodukt, vorzugsweise Sanitär- oder Hygiene-Ausgangsprodukt, oder zum Verbinden von jedem der zumindest teilweise mit einem vorzugsweise druckempfindlichen Klebstoff ausgerüsteten, vereinzelten Ausgangsprodukte, vorzugsweise Sanitär- oder Hygiene-Ausgangsprodukte, mit jeweils einem wenigstens eine lumineszenzfähige Verbindung enthaltenden, lumineszenzfähigen Silikonbeschichtungsabschnitt,
(5) ggf. eine Faltstation zum Falten jedes der nach (4) erhaltenen Produkte, vorzugsweise jedes der Sanitär- oder Hygiene-Produkte, und,
(6) ggf. eine Siegel-, und eine Stanz- und/oder Schneidevorrichtung zum Vereinzeln der nach (4) oder (5) erhaltenen Produkte, vorzugsweise Sanitär- oder Hygiene-Produkte, und,
(7) ggf. eine Verpackungsstation zur Verpackung jedes der nach (4), (5) oder (6) erhaltenen Produkte, vorzugsweise jedes Sanitär- oder Hygiene-Produktes,
(8) und ein Lumineszenz, vorzugsweise Fluoreszenz, empfindliches Sensorelement in direktem oder indirektem Kontakt mit
(9) einer Steuereinheit zur rapportgenauen Steuerung zumindest der beiden Fördermittel, der Dosiereinrichtung, der Umlenkvorrichtung, der Stanz- und/oder Schneidevorrichtung und ggf. der Verpackungsstation.

## Claims

1. Release film or protective film, whose silicone coating is respectively composed, at least at regular intervals, of a luminescent silicone coating section comprising at least one luminescent compound, **characterized in that** the design or form of the luminescent silicone coating sections corresponds to that of the product to be equipped with the release film or protective film, and the luminescent silicone coating sections comprise from 0.1 to 15% by weight, based on the total weight of the silicone coating, of the luminescent compound.

2. Release film or protective film according to Claim 1, **characterized in that** the silicone coating is composed only of the luminescent silicone coating sections comprising at least one luminescent compound and arranged at regular intervals.

3. Release film or protective film according to Claim 1 or 2, **characterized in that** each of the luminescent sections comprising at least one luminescent compound in the silicone coating is a fluorescent silicone section.

4. Release film or protective film according to any of the preceding claims, **characterized in that** each of the luminescent silicone sections of the silicone coating comprises at least one fluorescent compound, particularly preferably at least one fluorescent optical brightener, as luminescent compound.

5. Release film or protective film according to any of the preceding claims, **characterized in that** one of the surfaces of the release film or protective film has a silicone coating which is composed, at least at regular intervals, of a luminescent silicone coating section comprising at least one luminescent compound, and optionally, respectively in alternation, of a non-luminescent silicone coating section, or is composed only of luminescent coating sections comprising at least one luminescent compound and arranged at regular intervals.

6. Release film or protective film according to any of the preceding claims, **characterized in that** the luminescent sections of the silicone coating comprise from 0.5 to 10% by weight, based on the total weight of the silicone coating, of the luminescent compound.

7. Release film or protective film according to any of the preceding claims, **characterized in that** the release film or protective film comprises no luminescent markings, in particular no registration markings, control markings and/or print markings, other than the luminescent silicone coating sections comprising at least one luminescent compound.

8. Process for equipping a preferably sheet-like ingoing product, preferably an ingoing sanitary or hygiene product, with a release film or protective film according to any of Claims 1 to 7, comprising at least the following steps:
(a) synchronized, separate introduction of discrete ingoing products, preferably ingoing sanitary or hygiene products, and of the release film or protective film,
(b) at least partially equipping each of said luminescent silicone coating sections comprising at least one luminescent compound in the release film or protective film with a preferably pressure-sensitive adhesive, preferably in the form of at least one strip, or at least partially equipping each of the discrete ingoing products, preferably discrete sanitary or hygiene products, with a preferably pressure-sensitive adhesive, preferably in the form of at least one strip;
(c) bringing together and bonding respectively a luminescent silicone coating section at least partially equipped with a preferably pressure-sensitive adhesive and comprising at least one luminescent compound in the release film or protective film with respectively an ingoing product, preferably an ingoing sanitary or hygiene product, or bringing together and bonding respectively a discrete ingoing product at least partially equipped with a preferably pressure-sensitive adhesive, preferably ingoing sanitary or hygiene product, with respectively a luminescent silicone coating section comprising at least one luminescent compound in the release film or protective film,
(d) optional onward conveying of the products bonded with the release film or protective film, preferably sanitary or hygiene products,
(e) optional folding and optional sealing and separation of the products equipped with the release film or protective film, preferably sanitary or hygiene products,
(f) optional packaging of each product equipped with a release film or protective film, preferably sanitary or hygiene product, and optional further separation of the packaged products, preferably sanitary or hygiene products,
where at least the steps (b) and (c) and optionally also the step (a) are controlled with precise registration by at least one sensor element detecting luminescence, preferably fluorescence.

9. Process according to Claim 8, **characterized in that** the process is continuous.

10. Preferably sheet-like product, preferably a sanitary or hygiene product, obtainable by a process according to Claim 8 or 9.

11. Sanitary or hygiene product according to Claim 10, **characterized in that** the sanitary or hygiene product is a sanitary napkin or a pantyliner.

12. Apparatus for carrying out a process according to Claim 8 or 9, at least comprising:
(1) a first conveying means for conveying the discrete ingoing products, preferably ingoing sanitary or hygiene products,
(2) a second conveying means for conveying a release film or protective film according to any of Claims 1-7, whose silicone coating is respectively composed, at least at regular intervals, of a luminescent silicone coating section comprising at least one luminescent compound, where the design or form of the luminescent coating sections corresponds to that of the product to be equipped with the release film or protective film, and the luminescent silicone coating sections comprise from 0.1 to 15% by weight, based on the total weight of the silicone coating, of the luminescent compound,
(3) metering equipment for equipping, at least partially, each of the luminescent silicone coating sections with a preferably pressure-sensitive adhesive, or for equipping, at least partially, each discrete ingoing product, preferably discrete sanitary or hygiene product, with a preferably pressure-sensitive adhesive,
(4) a deflector apparatus for bringing the first conveying means together with the second conveying means and for bonding each luminescent silicone coating section at least partially equipped with a preferably pressure-sensitive adhesive and comprising at least one luminescent compound with a discrete ingoing product, preferably ingoing sanitary or hygiene product, or for bonding each of the discrete ingoing products at least partially equipped with a preferably pressure-sensitive adhesive, preferably ingoing sanitary or hygiene products, with respectively a luminescent silicone coating section comprising at least one luminescent compound,
(5) optionally a folding unit for folding each of the products obtained according to (4), preferably each of the sanitary or hygiene products, and
(6) optionally a sealing apparatus, and a punching apparatus, and/or cutting apparatus, for separating the products obtained according to (4) or (5), preferably sanitary or hygiene products, and
(7) optionally a packaging unit for packaging each of the products obtained according to (4), (5) or (6), preferably each sanitary or hygiene product,
(8) and a sensor element sensitive to luminescence, preferably fluorescence, in direct or indirect contact with
(9) a control unit for controlling, with precise registration, at least the two conveying means, the metering equipment, the deflection apparatus, the punching and/or cutting apparatus, and optionally the packaging unit.

## Revendications

1. Feuille de séparation ou de protection, dont le revêtement en silicone est au moins à intervalles réguliers constitué par un segment de revêtement en silicone luminescent contenant au moins un composé luminescent, **caractérisée en ce que** les segments de revêtement en silicone luminescents correspondent en configuration ou en forme au produit équipé de la feuille de séparation ou de protection, et contiennent 0,1 à 15 % en poids, par rapport au poids total du revêtement en silicone, du composé luminescent.

2. Feuille de séparation ou de protection selon la revendication 1, **caractérisée en ce que** le revêtement en silicone n'est constitué que par les segments de revêtement en silicone luminescents contenant au moins un composé luminescent, agencés à intervalles réguliers.

3. Feuille de séparation ou de protection selon la revendication 1 ou 2, **caractérisée en ce que** chacun des segments luminescents contenant au moins un composé luminescent du revêtement en silicone est un segment en silicone fluorescent.

4. Feuille de séparation ou de protection selon l'une quelconque des revendications précédentes, **caractérisée en ce que** chacun des segments en silicone luminescents du revêtement en silicone contiennent au moins un composé fluorescent, de manière particulièrement préférée au moins un azurant optique fluorescent, en tant que composé luminescent.

5. Feuille de séparation ou de protection selon l'une quelconque des revendications précédentes, **caractérisée en ce qu'**une des surfaces de la feuille de séparation ou de protection comprend un revêtement en silicone, qui est constitué au moins à intervalles réguliers par un segment de revêtement en silicone luminescent contenant au moins un composé luminescent et éventuellement en alternance par un segment de revêtement en silicone non luminescent ou uniquement par des segments de revêtement luminescents contenant au moins un composé luminescent, agencés à intervalles réguliers.

6. Feuille de séparation ou de protection selon l'une quelconque des revendications précédentes, **caractérisée en ce que** les segments luminescents du revêtement en silicone contiennent 0,5 à 10 % en poids, par rapport au poids total du revêtement en silicone, du composé luminescent.

7. Feuille de séparation ou de protection selon l'une quelconque des revendications précédentes, **caractérisée en ce que** la feuille de séparation et de protection ne comprend pas d'autres marques luminescentes en plus des segments de revêtement en silicone luminescents contenant au moins un composé luminescent, notamment pas de marques tactiles, de guidage et/ou d'impression.

8. Procédé d'équipement d'un produit de départ de préférence plat, de préférence d'un produit de départ sanitaire ou d'hygiène, avec une feuille de séparation ou de protection selon l'une quelconque des revendications 1 à 7, comprenant au moins les étapes suivantes :
(a) l'introduction séparée synchronisée de produits de départ individualisés, de préférence de produits de départ sanitaires ou d'hygiène, et de la feuille de séparation ou de protection,
(b) l'équipement au moins partiel de chacun de ces segments de revêtement en silicone luminescents contenant au moins un composé luminescent de la feuille de séparation ou de protection avec un adhésif de préférence sensible à la pression, de préférence sous la forme d'au moins une bande, ou l'équipement au moins partiel de chacun des produits de départ individualisés, de préférence des produits de départ sanitaires ou d'hygiène, avec un adhésif de préférence sensible à la pression, de préférence sous la forme d'au moins une bande ;
(c) la réunion et la liaison à chaque fois d'un segment de revêtement en silicone luminescent contenant au moins un composé luminescent, au moins partiellement équipé d'un adhésif de préférence sensible à la pression, de la feuille de séparation ou de protection avec à chaque fois un produit de départ, de préférence un produit de départ sanitaire ou d'hygiène, ou la réunion et la liaison à chaque fois d'un produit de départ individualisé au moins partiellement équipé d'un adhésif de préférence sensible à la pression, de préférence d'un produit de départ sanitaire ou d'hygiène, avec à chaque fois un segment de revêtement en silicone luminescent contenant au moins un composé luminescent de la feuille de séparation ou de protection,
(d) éventuellement le transport ultérieur des produits reliés avec la feuille de séparation ou de protection, de préférence des produits sanitaires ou d'hygiène,
(e) éventuellement le pliage, éventuellement le scellage, et l'individualisation des produits équipés de la feuille de séparation ou de protection, de préférence des produits sanitaires ou d'hygiène,
(f) éventuellement l'emballage de chaque produit équipé d'une feuille de séparation ou de protection, de préférence de chaque produit sanitaire ou d'hygiène, et éventuellement l'individualisation ultérieure des produits emballés, de préférence des produits sanitaires ou d'hygiène,
au moins les étapes (b) et (c) et éventuellement également l'étape (a) étant commandées avec grande précision par au moins un élément capteur enregistrant la luminescence, de préférence la fluorescence.

9. Procédé selon la revendication 8, **caractérisé en ce que** le procédé est continu.

10. Produit de préférence plat, de préférence produit sanitaire ou d'hygiène, pouvant être obtenu par un procédé selon la revendication 8 ou 9.

11. Produit sanitaire ou d'hygiène selon la revendication 10, **caractérisé en ce que** le produit sanitaire ou d'hygiène est une serviette hygiénique ou un protège-slip.

12. Dispositif pour la réalisation d'un procédé selon la revendication 8 ou 9, comprenant au moins :
(1) un premier moyen de transport pour le transport des produits de départ individualisés, de préférence des produits de départ sanitaires ou d'hygiène,
(2) un deuxième moyen de transport pour le transport d'une feuille de séparation ou de protection selon l'une quelconque des revendications 1 à 7, dont le revêtement de silicone est au moins à intervalles réguliers constitué par un segment de revêtement en silicone luminescent contenant au moins un composé luminescent, et les segments de revêtement en silicone luminescents correspondent en configuration ou en forme au produit à équiper avec la feuille de séparation ou de protection, et contiennent 0,1 à 15 % en poids, par rapport au poids total du revêtement en silicone, du composé luminescent,
(3) un dispositif de dosage pour l'équipement au moins partiel de chacun des segments de revêtement en silicone luminescents avec un adhésif de préférence sensible à la pression, ou pour l'équipement au moins partiel de chaque produit de départ individualisé, de préférence de chaque produit de départ sanitaire ou d'hygiène, avec un adhésif de préférence sensible à la pression,
(4) un dispositif de dérivation pour la réunion du premier moyen de transport avec le deuxième moyen de transport et pour la liaison de chaque segment de revêtement en silicone luminescent contenant au moins un composé luminescent, au moins partiellement équipé d'un adhésif de préférence sensible à la pression, avec un produit de départ individualisé, de préférence un produit de départ sanitaire ou d'hygiène, ou pour la liaison de chacun des produits de départ individualisés, au moins partiellement équipés avec un adhésif de préférence sensible à la pression, de préférence des produits de départ sanitaires ou d'hygiène, avec à chaque fois un segment de revêtement en silicone luminescent contenant au moins un composé luminescent,
(5) éventuellement une station de pliage pour le pliage de chacun des produits obtenus selon (4), de préférence de chacun des produits sanitaires ou d'hygiène, et
(6) éventuellement un dispositif de scellage, et un dispositif de poinçonnage et/ou de découpe, pour l'individualisation des produits obtenus selon (4) ou (5), de préférence des produits sanitaires ou d'hygiène, et
(7) éventuellement une station d'emballage pour l'emballage de chacun des produits obtenus selon (4), (5) ou (6), de préférence de chacun des produits sanitaires ou d'hygiène,
(8) et un élément capteur sensible à la luminescence, de préférence à la fluorescence, en contact direct ou indirect avec
(9) une unité de commande pour la commande de grande précision d'au moins les deux moyens de transport, le dispositif de dosage, le dispositif de dérivation, le dispositif de poinçonnage et/ou de découpe, et éventuellement la station d'emballage.
